# FASCICULE DE BREVET EUROPEEN

(11) **EP 1 321 471 B1**
(45) Date de publication et mention de la délivrance du brevet: **04.05.2005**
(21) Numéro de dépôt: 03290605.9
(22) Date de dépôt: 12.03.2003
(51) Int. Cl.: C07K 5/02, C07K 5/06, C07D 209/42

(54) **Nouveau procédé de synthèse du perindopril et de ses sels pharmaceutiquement acceptables**
Verfahren für die Synthese von Perindopril und seiner pharmazeutischen annehmbaren Salzen
Method for synthesis of perindopril and its pharmaceutically acceptable salts

(43) Date de publication de la demande: 25.06.2003
(73) Titulaire: LES LABORATOIRES SERVIER, 92415 Courbevoie Cédex (FR)
(72) Inventeur: Dubuffet, Thierry, 76210 Bolbec (FR); Lecouve, Jean-Pierre, 76600 Le Havre (FR)

(56) Documents cités:
- WO-A-01/58868
- WO-A-03/016336
- VINCENT M ET AL: "Stereoselective Synthesis of a New Perhydroindole Derivative of Chiral Iminodiacid, a Potent Inhibitor of Agiotensin Converting Enzyme" TETRAHEDRON, ELSEVIER SCIENCE PUBLISHERS, AMSTERDAM, NL, vol. 23, no. 16, 1982, pages 1677-1680, XP002155080 ISSN: 0040-4020

## Description

La présente invention concerne un procédé de synthèse industrielle du perindopril de formule (I): 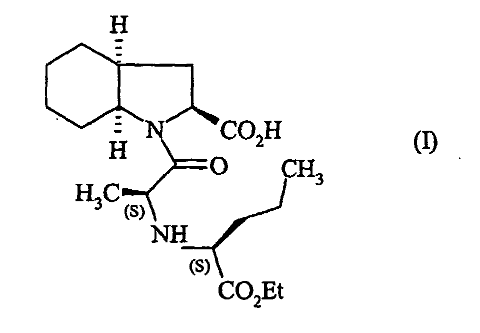 et de ses sels pharmaceutiquement acceptables.

Le perindopril, ainsi que ses sels pharmaceutiquement acceptables, et plus particulièrement son sel de tert-butylamine, possèdent des propriétés pharmacologiques intéressantes.
Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir y accéder avec un procédé de synthèse industrielle performant, facilement transposable à l'échelle industrielle, conduisant au perindopril avec un bon rendement et une excellente pureté, à partir de matières premières bon marché.
Le brevet EP 0 308 341 décrit la synthèse industrielle du perindopril par couplage de l'ester benzylique de l'acide (2S, 3aS, 7aS)-octahydroindole 2-carboxylique avec l'ester éthylique de la N-[(S)-1-carboxybutyl]-(S)-alanine, suivie de la déprotection du groupement carboxylique de l'hétérocycle par hydrogénation catalytique.
Or, l'ester de l'acide (2S, 3aS, 7aS)-octahydroindole-2-carboxylique n'est pas commercial, et sa préparation nécessite plusieurs étapes de synthèse (dont une étape de résolution) à partir de l'acide indole-2-carboxylique.

La demanderesse a présentement mis au point un nouveau procédé de synthèse du perindopril à partir de matières premières aisément accessibles.

Plus spécifiquement, la présente invention concerne un procédé de synthèse industrielle du perindopril, et de ses sels pharmaceutiquement acceptables, caractérisé en ce que l'on fait réagir la 2,7-oxepanedione de formule (II) : 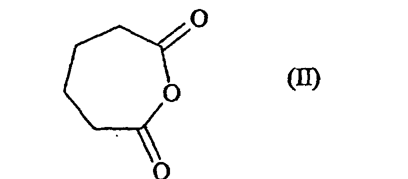 avec le composé de formule (III) : 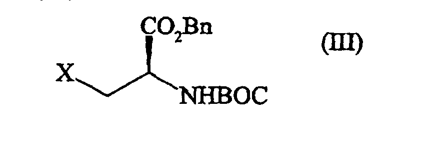 dans laquelle Bn représente le groupement benzyle, BOC représente le groupement tert-butyloxycarbonyle, et X représente un atome de brome ou d'iode,
en présence de zinc ou d'amalgame zinc/cuivre,
pour conduire après déprotection de la fonction aminé au composé de formule (IV) : 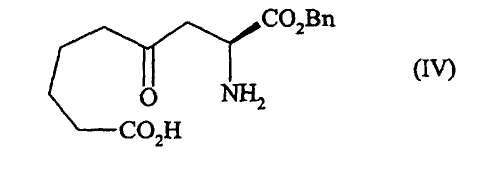 dans laquelle Bn est tel que défini précédemment,
que l'on cyclise par réaction avec un agent de chloration tel que le chlorure de thionyle ou le chlorure d'oxalyle, ou avec un agent de couplage peptidique, pour conduire au composé de formule (V) : 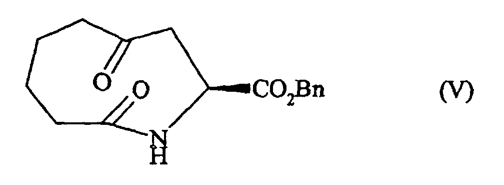 dans laquelle Bn est tel que défini précédemment,
que l'on soumet à une réaction de couplage en présence de titane,
pour conduire au composé de formule (VI) : 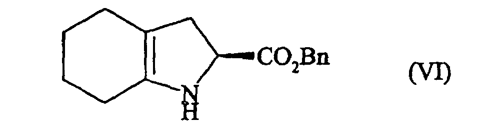 dans laquelle Bn est tel que défini précédemment,
que l'on met en réaction avec le composé de formule (VII) : 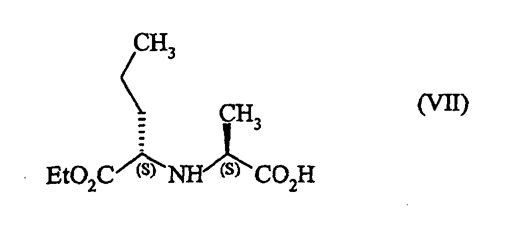 dans l'acétate d'éthyle,
en présence d'une quantité de 1-hydroxybenzotriazole comprise entre 0,4 et 0,6 mole par mole de composé de formule (VI) utilisé et d'une quantité de dicyclohexylcarbodiimide comprise entre 1 et 1,2 mole par mole de composé de formule (VI) utilisé,
en présence d'une quantité de triéthylamine comprise entre 0,25 et 1,2 mole par mole de composé de formule (VI) utilisé,
à une température comprise entre 20 et 77°C,
pour conduire après isolement au composé de formule (VIII) : 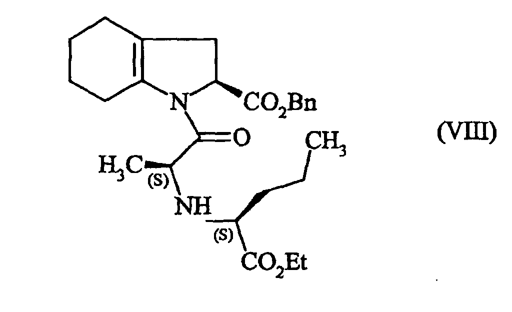 dans laquelle Bn est tel que défini précédemment,
que l'on hydrogène en présence d'un catalyseur tel que le palladium, le platine, le rhodium ou le nickel,
sous une pression d'hydrogène comprise entre 1 et 30 bars, de préférence entre 1 et 10 bars, pour conduire au perindopril de formule (I), que l'on transforme, si on le souhaite, en un sel pharmaceutiquement acceptable tel que le sel de tert-butylamine.

L'exemple ci-dessous illustre l'invention, mais ne la limite en aucune façon.

### EXEMPLE : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

### Stade A : Acide (8S)-9-benzyloxy-8-[(tert-butyloxycarbonyl)-amino]-6,9-dioxo-nonanoïque

Dans un réacteur, placer 200 g de 2,7-oxepanedione, 1 1 de diméthylformamide, 204 g de poudre de zinc puis 758 g de (2S)-2-[(tert-butyloxycarbonyl)-amino]-3-iodopropanoate de benzyle. Laisser la température du mélange réactionnel monter sous agitation à 60°C, puis maintenir cette température pendant 1 h. Ramener ensuite le mélange réactionnel à température ambiante, le filtrer puis le verser sur une solution glacée d'acide chlorhydrique à 5 %, puis extraire par l'acétate d'éthyle et évaporer à sec.
L'acide (8S)-9-benzyloxy-8-[(tert-butyloxycarbonyl)-amino]-6,9-dioxo-nonanoïque est ainsi obtenu avec un rendement de 80 %.

### Stade B : Acide (8S)-8-amino-9-benzyloxy-6,9-dioxo-nonanoïque

Dans un réacteur, placer 200 g du composé obtenu dans le stade précédent, 1,5 1 de dichlorométhane et 56 g d'acide trifluoroacétique. Après 1h30 d'agitation à température ambiante, ajouter 2 1 d'une solution saturée d'hydrogénocarbonate de sodium. Extraire par le dichlorométhane et évaporer à sec.
L'acide (8S)-8-amino-9-benzyloxy-6,9-dioxo-nonanoïque est ainsi obtenu avec un rendement de 90 %.

### Stade C : (2S)-4,9-Dioxo-2-azonanecarboxylate de benzyle

Dans un réacteur, placer 200 g du composé obtenu dans le stade précédent, 2 1 d'acétate d'éthyle puis 44 g de 1-hydroxybenzotriazole et 140 g de dicyclohexylcarbodiimide. Le mélange est ensuite porté à 30°C pendant 3 heures, puis il est refroidi et filtré. Le filtrat est ensuite lavé, puis évaporé à sec.

Le (2S)-4,9-dioxo-2-azonanecarboxylate de benzyle est ainsi obtenu avec un rendement de 95 %.

### Stade D: (2S)-2,3,4,5,6,7-Hexahydro-1H-indole-2-carboxylate de benzyle, paratoluènesulfonate

Dans un réacteur, placer 2 1 d'une suspension de titane sur graphite 0,7 M dans le tétrahydrofurane, puis amener au reflux et ajouter lentement une solution de 200 g du composé obtenu dans le stade précédent dans 2 1 de tétrahydrofurane. Amener ensuite le mélange réactionnel à température ambiante, le filtrer sur un lit de silice puis laver et évaporer le filtrat à sec. Reprendre le produit brut dans le toluène, ajouter 131 g d'acide paratoluènesulfonique monohydrate, porter la suspension au reflux et éliminer l'eau par distillation azéotropique. Refroidir le milieu à température ambiante, filtrer, laver le sel par du toluène puis le sécher.
Le paratoluènesulfonate du (2S)-2,3,4,5,6,7-hexahydro-1*H*-indole-2-carboxylate de benzyle est ainsi obtenu avec un rendement de 77 %.

### Stade E : (2S)-1-{(2S)-2-[(1S)-1-(Ethoxycarbonyl)-butylamino]-propionyl}-2,3,4,5,6,7-hexahydro-1H-indole-2-carboxylate de benzyle

Dans un réacteur, placer 200 g du composé obtenu dans le stade précédent, 46 g de triéthylamine, 1 1 d'acétate d'éthyle puis, après 10 mn d'agitation à température ambiante, 104 g de N-[(S)-carbéthoxy-1-butyl]-(S)-alanine, 30 g de 1-hydroxybenzotriazole et 100 g de dicyclohexylcarbodiimide. Le mélange hétérogène est ensuite porté à 30°C pendant 3 h sous bonne agitation, puis il est refroidi à 0°C et filtré. Le filtrat est ensuite lavé, puis évaporé à sec pour conduire au produit attendu avec un rendement de 95 %.

### Stade F: Acide (2S, 3aS, 7aS)-1-{(2S)-2-[(1S)-1-(éthoxycarbonyl)-butylamino]-propionyl}-octahydro-1H-indole-2-carboxylique

Dans un hydrogénateur, placer 200 g du composé obtenu dans le stade précédent, en solution dans l'acide acétique, puis 5 g de Pt/C à 10 %. Hydrogéner sous pression de 5 bars à température ambiante, jusqu'à absorption de la quantité théorique d'hydrogène.

Eliminer le catalyseur par filtration, puis refroidir entre 0 et 5°C et récolter le solide obtenu par filtration. Laver le gâteau et le sécher jusqu'à poids constant.
L'acide (2S, 3aS, 7aS)-1-{(2S)-2-[(1S)-1-(éthoxycarbonyl)-butylamino]-propionyl}-octahydro-1*H*-indole-2-carboxylique est ainsi obtenu avec un rendement de 85 % et une pureté énantiomérique de 99 %.

### Stade G : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxyl ique

Le composé obtenu dans le stade précédent (200 g) est mis en solution dans 2,8 l d'acétate d'éthyle, puis 40 g de tert-butylamine et 0,4 1 d'acétate d'éthyle sont ajoutés. La suspension obtenue est ensuite portée au reflux jusqu'à dissolution totale, puis la solution obtenue est filtrée à chaud et refroidie sous agitation jusqu'à une température de 15-20°C.
Le précipité obtenu est alors filtré, réempâté à l'acétate d'éthyle, séché puis broyé pour conduire au produit attendu avec un rendement de 95%.

## Revendications

1. Procédé de synthèse industrielle du composé de formule (I) : 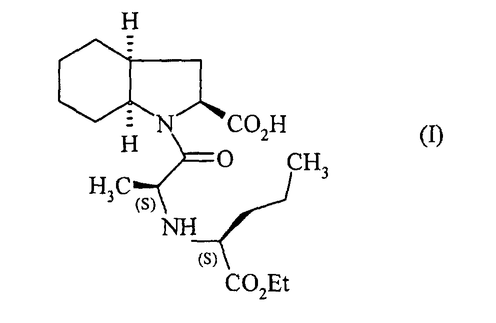 et de ses sels pharmaceutiquement acceptables,
**caractérisé en ce que** l'on fait réagir la 2,7-oxepanedione de formule (II): 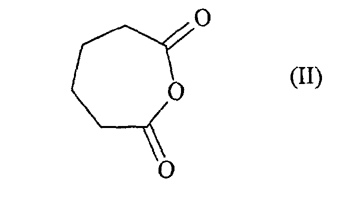 avec le composé de formule (III) : 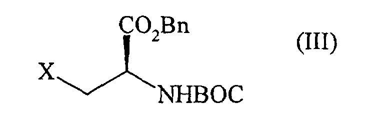 dans laquelle Bn représente le groupement benzyle, BOC représente le groupement tert-butyloxycarbonyle, et X représente un atome de brome ou d'iode,
en présence de zinc ou d'amalgame zinc/cuivre,
pour conduire après déprotection de la fonction amine au composé de formule (IV) : 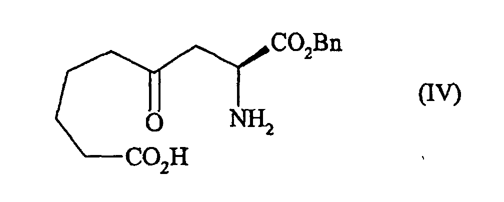 dans laquelle Bn est tel que défini précédemment,
que l'on cyclise par réaction avec un agent de chloration tel que le chlorure de thionyle ou le chlorure d'oxalyle, ou avec un agent de couplage peptidique, pour conduire au composé de formule (V) : 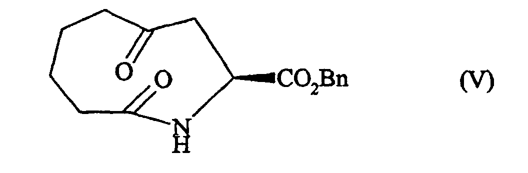 dans laquelle Bn est tel que défini précédemment,
que l'on soumet à une réaction de couplage en présence de titane,
pour conduire au composé de formule (VI) : 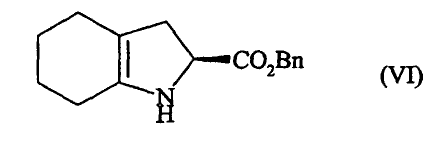 dans laquelle Bn est tel que défini précédemment,
que l'on met en réaction avec le composé de formule (VII) : 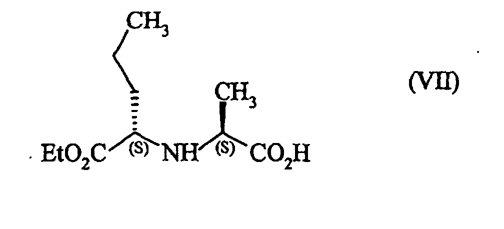 dans l'acétate d'éthyle,
en présence d'une quantité de 1-hydroxybenzotriazole comprise entre 0,4 et 0,6 mole par mole de composé de formule (VI) utilisé et d'une quantité de dicyclohexylcarbodiimide comprise entre 1 et 1,2 mole par mole de composé de formule (VI) utilisé,
en présence d'une quantité de triéthylamine comprise entre 0,25 et 1,2 mole par mole de composé de formule (VI) utilisé,
à une température comprise entre 20 et 77°C,
pour conduire après isolement au composé de formule (VIII) : 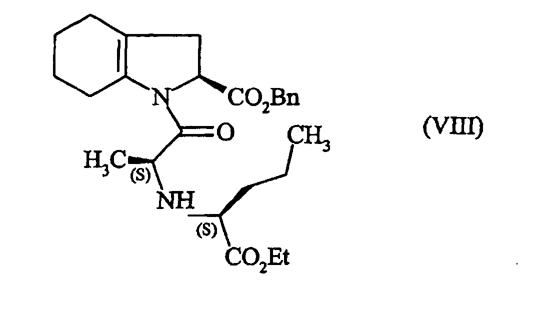 dans laquelle Bn est tel que défini précédemment,
que l'on hydrogène en présence d'un catalyseur tel que le palladium, le platine, le rhodium ou le nickel,
sous une pression d'hydrogène comprise entre 1 et 30 bars, pour conduire au perindopril de formule (I), que l'on transforme, si on le souhaite, en un sel pharmaceutiquement acceptable tel que le sel de tert-butylamine.

2. Procédé de synthèse selon la revendication 1, **caractérisé en ce que** la pression d'hydrogène de la réaction d'hydrogénation est comprise entre 1 et 10 bars.

3. Procédé de synthèse selon la revendication 1 du perindopril sous sa forme de sel de tert-butylamine.

## Patentansprüche

1. Verfahren zur technischen Synthese der Verbindung der Formel (I): 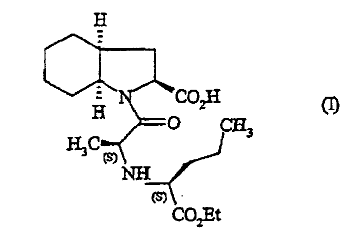 und deren pharmazeutisch annehmbaren Salzen,
**dadurch gekennzeichnet, daß** man 2,7-Oxepandion der Formel (II): 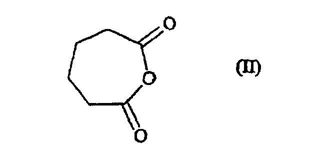 mit der Verbindung der Formel (III): 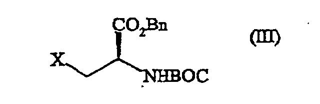 in der Bn die Benzylgruppe, BOC die tert-Butyloxycarbonyl-Gruppe und X ein Brom- oder Iodatom bedeuten,
in Gegenwart von Zink oder Zink/Kupfer-Amalgam umsetzt,
so daß man nach der Abspaltung der Schutzgruppe der Aminfunktion die Verbindung der Formel (IV) erhält: 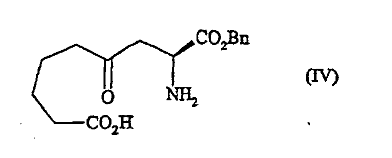 in der Bn die oben angegebene Bedeutung besitzt,
welche man durch Reaktion mit einem Chlorierungsmittel, wie Thionylchlorid, oder Oxalylchlorid, oder mit einem Peptid-Kupplungsmittel, cyclisiert, zur Bildung der Verbindung der Formel (V): 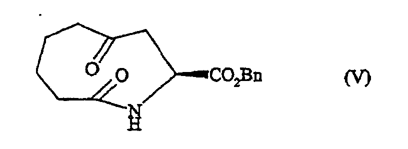 in der Bn die oben angegebene Bedeutung besitzt,
welche man einer Kupplungsreaktion in Gegenwart von Titan unterzieht,
zur Bildung der Verbindung der Formel (VI): 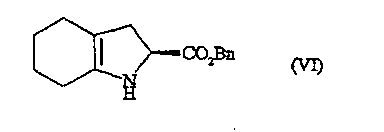 in der Bn die oben angegebene Bedeutung besitzt,
welche man mit der Verbindung der Formel (VII): 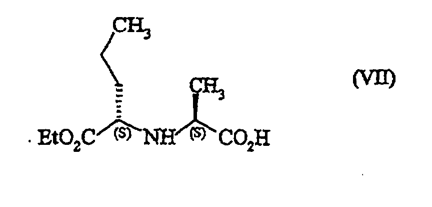 in Ethylacetat, in Gegenwart einer Menge von 1-Hydroxybenzotriazol zwischen 0,4 und 0,6 Mol pro Mol der verwendeten Verbindung der Formel (VI) und einer Menge von Dicyclohexylcarbodiimid zwischen 1 und 1,2 Mol pro Mol der verwendeten Verbindung der Formel (VI), in Gegenwart einer Menge Triethylamin zwischen 0,25 und 1,2 Mol pro Mol der verwendeten Verbindung der Formel (VI), bei einer Temperatur zwischen 20 und 77°C umsetzt,
so daß man nach der Isolierung die Verbindung der Formel (VIII) erhält: 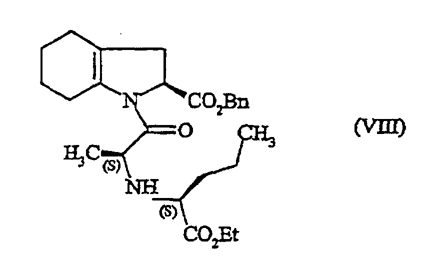 in der Bn die oben angegebene Bedeutung besitzt,
welche man in Gegenwart eines Katalysators, wie Palladium, Platin, Rhodium oder Nickel unter einem Wasserstoffdruck zwischen 1 und 30 bar hydriert, zur Bildung von Perindopril der Formel (I), welches man gewünschtenfalls in ein pharmazeutisch annehmbares Salz umwandelt, wie das tert-Butylaminsalz.

2. Syntheseverfahren nach Anspruch 1, **dadurch gekennzeichnet, daß** der Wasserstoffdruck bei der Hydrierreaktion zwischen 1 und 10 bar liegt.

3. Syntheseverfahren nach Anspruch 1 für die Herstellung von Perindopril in Form seines tert-Butylaminsalzes.

## Claims

1. Process for the industrial synthesis of the compound of formula (I) : 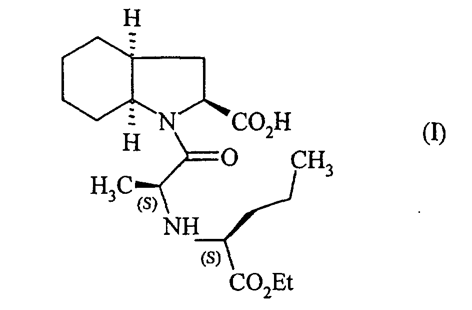 and pharmaceutically acceptable salts thereof,
**characterised in that** 2,7-oxepanedione of formula (II) : 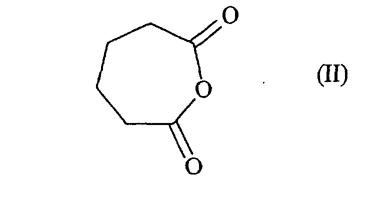 is reacted with the compound of formula (III) : 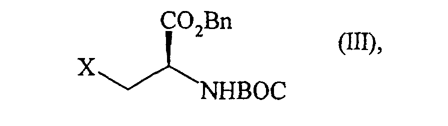 wherein Bn represents a benzyl group, BOC represents a tert-butoxycarbonyl group and X represents a bromine or iodine atom,
in the presence of zinc or zinc/copper amalgam,
to yield, after deprotection of the amine function, the compound of formula (IV) : 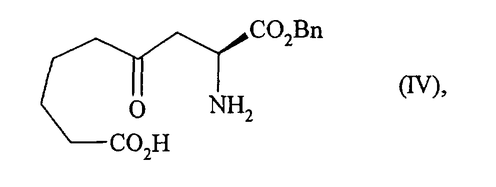 wherein Bn is as defined hereinbefore,
which is cyclised by reaction with a chlorination reagent such as thionyl chloride or oxalyl chloride or with a peptide coupling agent to yield the compound of formula (V) : 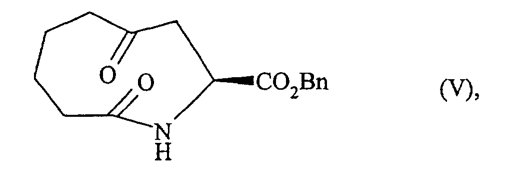 wherein Bn is as defined hereinbefore,
which is subjected to a coupling reaction in the presence of titanium
to yield the compound of formula (VI) : 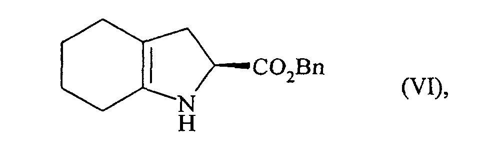 wherein Bn is as defined hereinbefore,
which is reacted with the compound of formula (VII) : 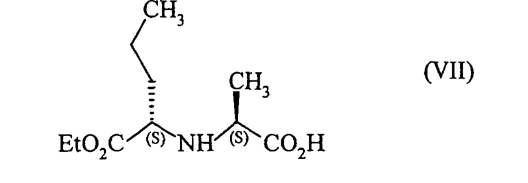 in ethyl acetate,
in the presence of an amount of 1-hydroxybenzotriazole of from 0.4 to 0.6 mol per mol of compound of formula (VI) used and an amount of dicyclohexylcarbodiimide of from 1 to 1.2 mol per mol of compound of formula (VI) used,
in the presence of an amount of triethylamine of from 0.25 to 1.2 mol per mol of compound of formula (VI) used,
at a temperature of from 20 to 77°C,
to yield, after isolation, the compound of formula (VIII) : 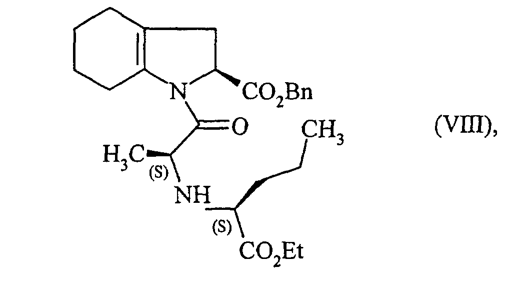 wherein Bn is as defined hereinbefore,
which is hydrogenated in the presence of a catalyst such as palladium, platinum, rhodium or nickel,
under a hydrogen pressure of from 1 to 30 bars, to yield perindopril of formula (I), which is converted, if desired, into a pharmaceutically acceptable salt such as the tert-butylamine salt.

2. Synthesis process according to claim 1, **characterised in that** the hydrogen pressure in the hydrogenation reaction is from I to 10 bars.

3. Process according to claim 1 for the synthesis of perindopril in the form of its tert-butylamine salt.
